(19) Europäisches Patentamt
European Patent Office
Office européen des brevets

(11) Veröffentlichungsnummer: **0 195 743**
**B1**

# (12) EUROPÄISCHE PATENTSCHRIFT

(45) Veröffentlichungstag der Patentschrift: **01.08.90**

(21) Anmeldenummer: **86810114.8**

(22) Anmeldetag: **05.03.86**

(51) Int. Cl.$^5$: **C 07 C 69/734,**
C 07 C 67/343, C 07 D 309/38

## (54) Enolether, Verfahren zu deren Herstellung, alpha-Pyrone, Verfahren zu deren Herstellung und Verwendung der alpha-Pyrone.

(30) Priorität: **11.03.85 CH 1089/85**

(43) Veröffentlichungstag der Anmeldung:
**24.09.86 Patentblatt 86/39**

(45) Bekanntmachung des Hinweises auf die Patenterteilung:
**01.08.90 Patentblatt 90/31**

(84) Benannte Vertragsstaaten:
**BE CH DE FR GB IT LI NL**

(56) Entgegenhaltungen:
**GB-A-2 156 808**

(73) Patentinhaber: **CIBA-GEIGY AG**
**Klybeckstrasse 141**
**CH-4002 Basel (CH)**

(72) Erfinder: **Kvita, Vratislav, Dr.**
**Vogesenstrasse 71**
**CH-4153 Reinach (CH)**
Erfinder: **Mayer, Carl W., Dr.**
**Steingrubenweg 224**
**CH-4125 Riehen (CH)**

**EP 0 195 743 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft Alkoxymethylenaconitsäuretriester, ein Verfahren zu deren Herstellung, 2-Oxo-2H-Pyran-4,5-dicarbonsäurediester (im folgenden auch α-Pyron-4,5-dicarbonsäurediester genannt), ein Verfahren zu deren Herstellung und deren Verwendung als Dienkomponente in diels-Alder-Reaktionen.

Es ist bekannt (vergleiche W. Lehnert, Tetrahedron Letters *54,* 4723—4724 (1970), Tetrahedron *28,* 663—666 (1972), Tetrahedron *29,* 635—638 (1973), Tetrahedron *30,* 301—305 (1974)), dass einige aliphatische Aldehyde und Ketone mit CH-aciden Verbindungen, z.B. Malonester, in Gegenwart von $TiCl_4$, einem Ether und einem tertiären Amin in einer Knoevenagel-Kondensation zu den entsprechenden Alkyliden-Verbindungen reagieren.

Weiterhin ist aus N. P. Shusherina, Russian Chemical Reviews *43* (10), 1974, S. 851—861, bekannt, dass z.B. 2-Oxo-2H-Pyran-5-carbonsäuremethylester als Dienkomponente in Diels-Alder-Reaktionen mit unterschiedlichen Dienophilen umgesetzt werden kann. α-Pyrone mit zwei Estergruppen in 4- und 5-Stellung sind nicht bekannt. Es ist jedoch wünschenswert, diese Verbindungen bereitzustellen, weil sie aufgrund der beiden funtktionellen Gruppen in vielfältiger Weise als Zwischenprodukte verwendet werden können.

Gegenstand der vorliegenden Erfindung sind Alkoxymethylenaconitsäuretriester der Formel I

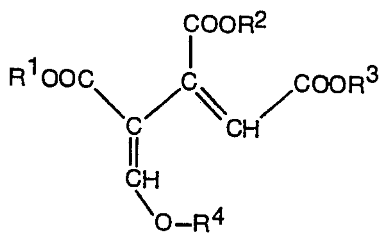

(I),

worin $R^1$, $R^2$, $R^3$ und $R^4$ unabhängig voneinander unsubstituierte oder substituierte aliphatische Kohlenwasserstoffreste darstellen.

$R^1$, $R^2$, $R^3$ und $R^4$ als Kohlenwasserstoffreste enthalten unabhängig voneinander bevorzugt 1 bis 20, besonders 1 bis 12 C-Atome.

Die Reste $R^1$, $R^2$, $R^3$ und $R^4$ stellen unabhängig voneinander bevorzugt unsubstituiertes oder substituiertes lineares oder verzweigtes Alkyl, Cycloalkyl, Alkylcycloalkyl, Aralkyl oder Alkaralkyl dar.

$R^1$, $R^2$, $R^3$ und $R^4$ enthalten als Alkyl unabhängig voneinander bevorzugt 1 bis 18 C-Atome, besonders 1 bis 12 und insbesondere 1 bis 6 C-Atome.

Beispiele für Alkyl sind Methyl, Ethyl, n-Propyl, iso-Propyl, n-, iso- und tertiär-Butyl, Pentyl, Hexyl, Octyl, 2-Ethylhexyl, Nonyl, Decyl, Undecyl, Dodecyl, Tetradecyl, Octadecyl.

In einer besonderen Ausführungsform stellen $R^1$, $R^2$, $R^3$ und $R^4$ unabhängig voneinander Methyl oder Ethyl dar.

$R^1$, $R^2$, $R^3$ und $R^4$ als Cycloalkyl können unabhängig voneinander 3 bis 12, vorzugsweise 3 bis 8 und insbesonder 4 bis 6 Ringkohlenstoffatome enthalten.

Beispiele sind Cyclopropyl, Cyclobutyl, Cyclopentyl, Cyclohexyl, Cyclohepty, Cyclooctyl, Cyclononyl, Cyclodecyl, Cyclododecyl.

Bevorzugt sind Cyclopentyl oder Cyclohexyl.

$R^1$, $R^2$, $R^3$ und $R^4$ als Alkylcycloalkyl haben unabhängig voneinander bevorzugt 4 bis 24 C-Atome.

Beispiele sind die zuvor genannten, durch lineares oder verzweigtes Alkyl, vorzugsweise durch Methyl, Ethyl oder Propyl, substituierten Cycloaliphaten.

$R^1$, $R^2$, $R^3$ und $R^4$ als Aralkyl und Alkaralkyl enthalten unabhängig voneinander ein- oder mehrkernige, auch kondensierte aromatische Kohlenwasserstoffreste. Bevorzugt enthalten das Aralkyl 7 bis 15 C-Atome und das Alkaralkyl 8 bis 15 C-Atome.

Bei dem aromatischen Kohlenwasserstoffrest im Aralkyl und Alkaralkyl handelt es sich bevorzugt um Phenyl oder Naphthyl.

Beispiele sind Benzyl, Phenylethyl, 2-Phenylpropyl, Naphthylmethyl, Methylbenzyl, Ethylbenzyl.

Die aliphatischen Kohlenwasserstoffreste $R^1$, $R^2$, $R^3$ und $R^4$ können unabhängig voneinander ein- oder mehrfach mit gleichen oder verschiedenen Gruppen substituierte sein, bevorzugt ein- bis dreifach. Geeignete Substituenten für $R^1$ bis $R^4$ sind solche, die unter den angegebenen Reaktionsbedingungen nicht mit $TiCl_4$ reagieren.

Beispiele sind Nitril, Halogen, besonders F, Cl, Br und insbesondere F und Cl, und Alkoxy. Alkoxy-Substituenten enthalten bevorzugt 1 bis 12, besonders 1 bis 4 C-Atome. In einer bevorzugten Ausführungsform sind $R^1$ bis $R^4$ mit einer Alkoxy- oder Nitrilgruppe oder ein oder mehrfach mit Halogen substituiert.

Als Halogen-substituierte Gruppen stellen die Reste $R^1$, $R^2$, $R^3$ und $R^4$ unabhängig voneinander vorzugsweise Halogenalkyl dar, welches in α-Stellung nicht Halogen-substituiert ist.

$R^1$, $R^2$, $R^3$ und $R^4$ als Halogenalkyl enthalten unabhängig voneinander bevorzugt 2 bis 6 C-Atome.

Beispiele sind 1-Chloreth-2-yl, 1-Brometh-2-yl, 1-Fluoreth-2-yl, 2-Chlorprop-2-yl, 1,1-Dichloreth-2-yl, 1,1-Difluoreth-2-yl, 1,1,1-Trichloreth-2-yl, 1,1,1-Trichlorprop-3-yl, 1,1,1-Trifluoreth-2-yl, 1,1,1-Trifluor-2,2-dichlorprop-3-yl.

In einer besonderen Ausführungsform der Enolether gemäss Formel I sind die Reste $R^1$, $R^2$, $R^3$ und $R^4$ identisch.

Die erfindungsgemässen Verbindungen können nach einem neuen Verfahren durch die Umsetzung eines Ameisensäureesters mit einem Aconitsäuretriester unter sehr milden Reaktionsbedingungen in einfacher Weise und mit hohen Ausbeuten erhalten werden.

Ein weiterer Gegenstand der vorliegenden Erfindung ist ein Verfahren zur Herstellung von Alkoxymethylenaconitsäuretriestern der Formel I mittels Reaktion von Carbonylverbindungen mit CH-aciden Bindungen und einem Ester in Gegenwart von $TiCl_4$, einem tertiären Amin und einem komplexbildenden Lösungsmittel, das dadurch gekennzeichnet ist, dass als Carbonylverbindung Aconitsäuretriester der Formel II

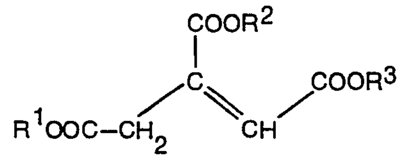

(II),

worin $R^1$, $R^2$ und $R^3$ unabhängig voneinander unsubstituierte oder substituierte aliphatische Kohlenwasserstoffreste darstellen, und als Ester Ameisensäureester der Formel III

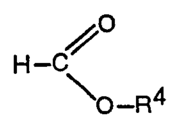

(III),

worin $R^4$ unabhängig die gleiche Bedeutung wie $R^1$ hat, zum Enolether der Formel I umgesetzt werden.

Ueberraschenderweise verhält sich der Ameisensäureester bei der Reaktion wie ein Aldehyd und bildet mit dem Aconitsäuretriester unter formaler Wasserabspaltung einen Enolether.

Die Reaktion wird vorzugsweise bei einer Temperatur von −20 bis +50°C, besonders bei 0 bis +30°C, durchgeführt, wobei die Reaktionszeiten einige Stunden betragen können.

Die Verbindungen der Formel III sind teilweise kommerziell erhältlich, oder können nach an sich bekannten Methoden durch Umesterung aus den käuflichen Ameisensäureestern hergestellt werden. Die Verbindungen der Formel II sind bekannt und können z.B. durch Wasserabspaltung aus Zitronensäureester hergestellt werden.

Die Ameisensäureester der Formel III können in äquimolarer Menge oder in einem Ueberschuss, bezogen auf den Aconitsäuretriester der Formel II, eingesetzt werden. Der Ueberschuss kann so gewählt werden, dass der Ameisensäureester gleichzeitig als Lösungsmittel dient. Zweckmässig ist ein 2- bis 8-facher, insbesondere 2- bis 5-facher Ueberschuss.

In einer bevorzugten Ausführungsform des Verfahrens sind die aliphatischen Kohlenwasserstoffreste $R^3$ und $R^4$ unabhängig voneinander $C_1$—$C_6$-Alkyl, insbesondere Methyl oder Ethyl.

Eine besonders vorteilhafte Ausführungsform des Verfahrens ist dadurch gekennzeichnet, dass die Reste $R^1$, $R^2$, $R^3$ und $R^4$ gleich sind, da eine unter den gegebenen Reaktionsbedingungen mögliche Umesterung vermieden und damit ein einheitliches Umsetzungsprodukt erhalten wird.

Das tertiäre Amin kann z.B. ein mono-, di- oder tri-Amin sein, dessen N-Atome alkyliert sind.

Bevorzugte tertiäre Amine sind Amine der Formel IV

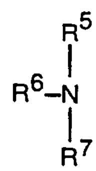

(IV),

worin $R^5$, $R^6$ und $R^7$ unabhängig voneinander verzweigtes oder unverzweigtes Alkyl mit vorzugsweise 1 bis 20, insbesondere 1 bis 12 C-Atomen; Aralkyl oder Aryl mit vorzugsweise 7 bis 15 bzw. 6 bis 15 C-Atomen; oder Cycloalkyl mit bevorzugt 4 bis 7 Ringkohlenstoffatomen.

Das Aralkyl ist bevorzugt Benzyl und das Aryl bevorzugt Phenyl.

Weitere geeignete tertiäre Amine sind heteroaromatische oder aliphatisch-heterocyclische Amine, wobei aliphatisch-heterocyclische Amine N-alkyliert, besonders N-methyliert, sind. Die heteroaromatischen Amine enthalten bevorzugt 5 oder 6 Ringglieder.

Die aliphatisch-heterocyclischen Amine weisen bevorzugt 3 bis 7, besonders 5 bis 7 Ringglieder auf. Sie können weitere Heteroatome, besonders N, O und S, enthalten.

Beispiele sind Trimethylamin, Triethylamin, Methyl-diethylamin, Tri-n-propylamin, Triisopropylamin, Methyl-diisopropylamin, Tri-n-butylamin, Tri-isobutylamin, Tri-tertiär-butylamin, Trihexylamin, Dimethylcyclohexylamin, Pyridin, Chinolin, Diamine der allgemeinen Formel $(Alkyl)_2—N—(CH_2)_n—N(Alkyl)_2$ mit n = 2 bis 6, N-Alkyl-piperidin, N-alkyl-pyrrolidin, N-Alkyl-morpholin, N-Alkyl-pyrazolin, N,N'-Dialkylpiperazin, wobei das Alkyl 1 bis 4 C-Atome enthält und besonders Methyl ist.

Besonders vorteilhaft ist die Verwendung von N-Methyl-morpholin als tertiäres Amin.

Das tertiäre Amin kann in äquimolarer Menge oder im Ueberschuss, bezogen auf den Aconitsäuretriester, eingesetzt werden. Der Ueberschuss kann so gewählt werden, dass das tertiäre Amin gleichzeitg als Lösungsmittel dient. Zweckmässig ist ein 2- bis 20-facher, insbesondere ein 4- bis 12-facher Ueberschuss.

Das $TiCl_4$ kann in etwa äquimolarer Menge oder im geringen Ueberschuss, bezogen auf den Ameisensäureester, eingesetzt werden. Bevorzugt setzt man äquimolare Mengen ein.

Vom komplexbildenden Lösungsmittel sind vorteilhaft mindestens 2 Mol pro Mol $TiCl_4$ zugegen. Das komplexbildende Lösungsmittel kann gleichzeitig Lösungsmittel für die Reaktion sein.

Geeignete, für $TiCl_4$ komplexbildende Lösungsmittel sind z.B. Stickstoff-, Sauerstoff- oder Schwefel-Atome enthaltende Lösungsmittel, die ihre Heteroatome über freie Elektronenpaare mit dem Titan koordinieren können, wobei bevorzugt Ether, besonders aliphatische Ether verwendet werden.

Beispiele sind Diethylether, Di-n-propylether, Di-isopropylether, Di-n-butylether, Di-iso-butylether, Di-tert.-butylether, Dihexylether, Tetrahydrofuran, Dioxan, Glycolether der allgemeinen Formel $R^8—O—(CH_2)_n—O—R^8$ mit n = 2 bis 6 und $R^8$ gleich $C_1—C_4$-Alkyl, besonders Methyl oder Ethyl, z.B. Ethylenglycoldimethylether oder Ethylenglycoldiethylether.

Weitere geeignete Ether sind z.B. Polyalkylenglycoldiether, die der Formel $R^8O—(C_nH_{2n}—O)_x—R^8$ entsprechen können, worin $R^8$ $C_1—C_4$-Alkyl, besonders Methyl oder Ethyl ist, n für 2—6, bevorzugt 2—4 und x für 2—4 stehen. Beispiele hierfür sind Diethylenglycoldimethylether, Triethylenglycoldimethylether, Dipropylenglycoldimethylether und Dibutylenglycoldiethylether.

Eine bevorzugte Ausführungsform des Verfahrens ist dadurch gekennzeichnet, dass die Reaktion in Gegenwart eines zusätzlichen inerten Lösungsmittels, insbesondere eines polaren aprotischen Lösungsmittels, ausgeführt wird. Beispiele sind halogenierte Kohlenwasserstoffe wie Methylenchlorid, Chloroform, Tetrachlorkohlenstoff, 1,2-Dichlorethan, 1,1,1-Trichlorethan, 1,1,2,2-Tetrachlorethan oder substituierte Benzole wie Chlorbenzol, Toluol oder Xylol.

Die Verbindungen der Formel I sind wertvolle polyfunktionelle Zwischenprodukte für die Synthese organischer Verbindungen. Sie können beispielsweise zu α-Pyron-4,5-dicarbonsäurediestern cyclisiert werden.

Ein weiterer Gegenstand der vorliegenden Erfindung sind α-Pyron-4,5-dicarbonsäurediester der Formel V

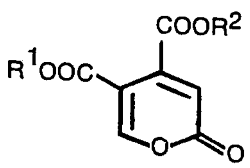

(V),

worin $R^1$ und $R^2$ unabhängig voneinander die gleiche Bedeutung wie in Formel I haben.

Für $R^1$ und $R^2$ gelten hierbei die zuvor erwähnten Bevorzugungen.

Die erfindungsgemässen Verbindungen können durch Cyclisierung von Alkoxymethylenaconitsäuretriester der Formel I in an sich bekannter Weise erhalten werden.

Ein weiterer Gegenstand der Erfindung ist ein Verfahren zur Herstellung von α-Pyronen der Formel V, dadurch gekennzeichnet, dass man einen Alkoxymethylenaconitsäuretriester der Formel I

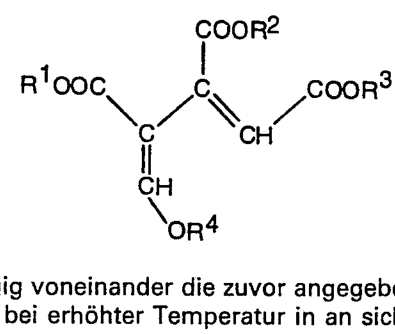

(I),

worin $R^1$, $R^2$, $R^3$ und $R^4$ unabhängig voneinander die zuvor angegebene Bedeutung haben, in Gegenwart einer starken wasserfreien Säure bei erhöhter Temperatur in an sich bekannter Weise cyclisiert und die

erhaltene Verbindung der Formel V in an sich bekannter Weise isoliert.

Bei der Reaktion werden starke wasserfreie Säuren, bevorzugt organische Säuren, und Temperaturen von bevorzugt 50—200°C, besonders 70—170°C, angewendet. Beispiele für organische Säuren sind Ameisensäure, Essigsäure, Propionsäure, Buttersäure, Fluorsulfonsäure, Chlorsulfonsäure, Methansulfonsäure, Toluolsulfonsäure, Benzolsulfonsäure. Die Reaktion wird vorzugsweise in Ameisensäure oder Essigsäure und ohne Zusatz eines Lösungsmittels durchgeführt.

Die Verbindungen der Formel V sind wertvolle polyfunktionelle Zwischenprodukte für die Synthese organischer Verbindungen. Sie können beispielsweise als Dienkomponenten in Diels-Alder-Reaktionen eingesetzt werden, wobei unterschiedliche Dienophile verwendet werden könne. Sie lassen sich hierbei, verglichen mit dem α-Pyronmonocarbonsäuremethylester, unter milderen Reaktionsbedingungen umsetzen. Aufgrund der vorhandenen funktionellen Gruppe können solche diels-Alder-Addukte z.B. in polycyclische Verbindungen umgewandelt werden.

Ein weiterer Gegenstand der vorliegenden Erfindung betrifft die Verwendung der 2-Oxo-2H-Pyran-4,5-dicarbonsäurediester der Formel V als Dienkomponente in Diels-Alder-Reaktionen.

Die nachfolgenden Beipiele erläutern die Erfindung näher.

Beispiel 1

Herstellung von α-Pyron-4,5-dicarbonsäurediethylester

In 1200 ml Tetrahydrofuran wird bei 2—3°C innerhalb 40 Minuten eine Lösung von 113,14 g (0,6 Mol) Titantetrachlorid in 150 ml Tetrachlormethan unter Rühren zugetropft. Nach einer Stunde werden bei derselben Temperatur nacheinander 44,16 g (0,6 Mol) Ameisensäureethylester und 38,7 g (0,15 Mol) Aconitsäuretriethylester zugefügt. Schliesslich wird noch während 45 Minuten 120,93 g (1,2 Mol) N-Methylmorpholin in 210 ml Tetrahydrofuran zugegeben. Nach 15 Minuten wird das Reaktionsgemisch in 1500 ml $H_2O$, 200 g Natriumbicarbonat und 1400 ml Methylenchlorid unter intensivem Rühren eingegossen. Es wird gerührt, bis sich kein $CO_2$ mehr entwickelt. Die entstandene Suspension wird zuerst abfiltriert und die beiden Phasen des Filtrats abgetrennt. Die wässrige Phase wird noch zweimal mit jeweils 300 ml Methylenchlorid ausgeschüttelt. Die vereinten Methylenchlorid-Extrakte werden mit Natriumsulfat getrocknet und eingeengt. Aus dem auf solche Weise erhaltenen Rohprodukt (44,95 g, 95,4%) werden bei 61°C im Hochvakuum nichtreagierte Bestandteile abdestilliert. Man erhält als Destillationsrückstand 31,38 g (66,6%) Ethoxymethylenaconitsäuretriethylester. $^1H$—NMR-Spektrum (in $CDCl_3$): 6,85 (1H, Singulett), 7,5 (1H, Singulett). Das Produkt wird direkt weiter verarbeitet.

31,38 g (0,1 Mol) Ethoxymethylenaconitsäuretriethylester werden in 314 ml Ameinsensäure bei 95°C 1 Stunde erwärmt. Die Ameisensäure wird im Vakuum der Wasserstrahlpumpe abdestilliert. Durch Destillation des Rückstandes im Hochvakuum erhält man 10,00 g (62,4%) α-Pyron-4,5-dicarbonsäurediethylester (Kp. 82°C/1,2 × $10^{-2}$ mbar). $^1H$-NMR-Spektrum (in $CDCl_3$): 6,40 (1H, Singulett), 8,25 (1H, Singulett).

Anwendungsbeispiele

Beispiel 2

Herstellung von Anthracen-9,10-chinon-2,3-dicarbonsäurediethylester

45,79 g (0,1908 mol) α-Pyron-4,5-dicarbonsäurediethylester und 25,15 g (0,1590 mol) Naphthochinon werden in 150 ml Xylol 17 Stunden unter Rühren am Rückfluss gehalten. Das zunächst entstehende Hydrochinon ist im verwendeten Reaktionsmedium an der Luft instabil und oxidiert spontan zum Chinon.

Das Xylol wird abdestilliert, und der Destillationsrückstand in 300 ml Methanol heiss gelöst und auf 20°C abgekühlt. Das kristalline rohe Produkt wird abgesaugt, in 300 ml Chloroform gelöst und über 100 g Kieselgel filtriert. Man erhält 20,73 (37%) Anthracen-9,10-chinon-2,3-dicarbonsäurediethylester, Smp. 153—155°C (aus Ethanol), $^1H$—NMR-Spektrum ($CDCl_3$): 8,88 (Singulett), 8,40 (Multiplett), 7,73 (Multiplett).

Beispiel 3

Herstellung von Naphthacen-5,12-chinon-2,3-dicarbonsäurediethylester

5,97 g (0,076 Mol) 1,4-Anthrachinon und 18,42 g (0,076 Mol α-Pyron-4,5-dicarbonsaurediethylester werden in 34 ml Xylol im Autoklaven 24 Stunden auf 160°C erhitzt. Xylol wird abdestilliert, der Destillationsrückstand in Chloroform gelöst, über 1,2 kg Kieselgel (Korngrösse: 0,040—0,063 mm) filtriert, und das Kieselgel mit Chloroform durchgespült. Man erhält 19,7 g rohe dunkelbraune Substanz, die einer Sublimation bei 220°C im Hochvakuum unterzogen wird. Man erhält 6,3 g Naphthacen-5,12-chinon-2,3-dicarbonsäurediethylester (30%), Smp. 208—209°C, Massenspektrum: $M^+ = 402$. Mit der Rückgewinnung des unreagierten α-Pyron-4,5-dicarbonsäure-diethylesters ist die Ausbeute quantitativ.

Beispiel 4

Herstellung von Naphthalen-1,4-chinon-6,7-dicarbonsäurediethylester

2,4 g (0,01 Mol) α-Pyron-4,5-dicarbonsäurediethylester und 5,4 g (0,05 Mol) 1,4-Benzochinon werden in 7 ml 1,2-Dichlorbenzol 12 Stunden unter Rühren am Rückflüss gehalten und dann 5 Stunden bei Zimmertemperatur stehengelassen. Die gebildeten schwarzen Kristalle werden mit 3 ml Dichlorbenzol nachgewaschen und die vereinte dunkle Dichlorbenzol-Lösung wird über 36 g Silikagel chromatographiert

(Laufmittel: Methylenchlorid). Die Methylenchlorid-Lösung wird bis zur Trockene am Rotationsverdampfer eingedampft. Bei 90°C Wasserbadtemperatur sublimiert dabei das überschüssige 1,4-Benzochinon. Aus dem öligen Destillationsrückstand kristallisiert nach Zugabe von 1 ml Ethyläther 1,1 g (36,4%) Smp. 59—62°C des Naphthalen-1,4-chinon-6,7-dicarbonsäure-diethylesters. Ein weiterer Anteil kann aus den Mutterlaugen gewonnen werden. $^1H$—NMR-Spektrum ($CDCl_3$): 8,42 (Singulett), 5,06 (Singulett). Massenspektrum: $M^+ = 302$.

**Patentansprüche**

1. Alkoxymethylenconitsäuretriester der Formel I

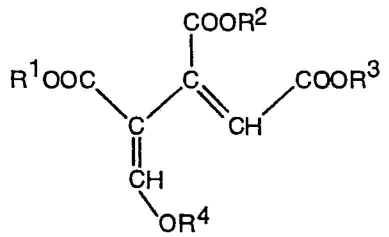

(I),

worin $R^1$, $R^2$, $R^3$ und $R^4$ unabhängig voneinander unsubstituierte oder substituierte aliphatische Kohlenwasserstoffreste darstellen.

2. Ester gemäss Anspruch 1, dadurch gekennzeichnet, dass die Reste $R^1$, $R^2$, $R^3$ und $R^4$ unabhängig voneinander lineares oder verzweigtes Alkyl, Cycloalkyl, Alkylcycloalkyl, Aralkyl und Alkaralkyl sind.

3. Ester gemäss Anspruch 2, dadurch gekennzeichnet, dass die Reste $R^1$, $R^2$, $R^3$ und $R^4$ unabhängig voneinander $C_1$—$C_{18}$-Alkyl, $C_3$—$C_{12}$-Cycloalkyl, $C_4$—$C_{24}$-Alkylcycloalkyl, $C_7$—$C_{15}$-Aralkyl oder $C_8$—$C_{15}$-Alkaralkyl darstellen.

4. Ester gemäss Anspruch 1, dadurch gekennzeichnet, dass die aliphatischen Kohlenwasserstoffreste $R^1$, $R^2$, $R^3$ und $R^4$ unabhängig voneinander mit einer Alkoxy- oder Nitril-Gruppe oder ein- oder mehrfach mit Halogen substituiert sind.

5. Ester gemäss Anspruch 1, dadurch gekennzeichnet, dass die aliphatischen Kohlenwasserstoffreste $R^1$, $R^2$, $R^3$ und $R^4$ unabhängig voneinander $C_1$—$C_6$-Alkyl bedeuten.

6. Ester gemäss Anspruch 1, dadurch gekennzeichnet, dass die aliphatischen Kohlenwasserstoffreste $R^1$, $R^2$, $R^3$ und $R^4$ gleich sind.

7. Ester gemäss Anspruch 1, dadurch gekennzeichnet, dass die aliphatischen Kohlenwasserstoffreste $R^1$, $R^2$, $R^3$ und $R^4$ unabhängig voneinander Methyl oder Ethyl darstellen.

8. Verfahren zur Herstellung von Alkoxymethylenaconitsäuretriester der Formel I gemäss Anspruch 1 mittels Reaktion von Carbonylverbindungen mit CH-aciden Bindungen und einem Ester in Gegenwart von $TiCl_4$, einem tertiären Amin und einem komplex-bildenden Lösungsmittel, dadurch gekennzeichnet, dass als Carbonylverbindung Aconitsäuretriester der Formel II

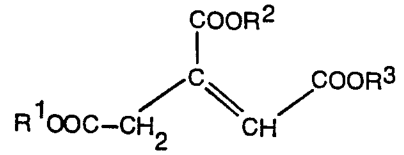

(II),

worin $R^1$, $R^2$ und $R^3$ unabhängig voneinander unsubstituierte oder substituierte aliphatische Kohlenwasserstoffreste darstellen und als Ester Ameisensäureester der Formel III

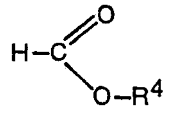

(III),

worin $R^4$ unabhängig die gleiche Bedeutung wie $R^1$ hat, umgesetzt werden.

9. Verfahren gemäss Anspruch 8, dadurch gekennzeichnet, dass die Reaktion bei einer Temperatur von −20°C bis +50°C ausgeführt wird.

10. Verfahren gemäss Anspruch 8, dadurch gekennzeichnet, dass das tertiäre Amin ein Amin der Formel IV

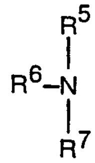

(IV),

worin $R^5$, $R^6$ und $R^7$ unabhängig voneinander verzweigtes oder unverzweigtes Alkyl, Cycloalkyl, Aralkyl oder Aryl bedeuten, oder ein hetero-aromatisches oder aliphatische-heterocyclisches Amin, wobei aliphatisch-heterocyclische Amine N-alkyliert sind, ist.

11. Verfahren gemäss Anspruch 10, dadurch gekennzeichnet, dass das tertiäre Amin N-Methylmorpholin ist.

12. Verfahren gemäss Anspruch 8, dadurch gekennzeichnet, dass das komplexbildende Lösungsmittel ein Ether ist.

13. Verfahren gemäss Anspruch 8, dadurch gekennzeichnet, dass bei der Reaktion zusätzlich ein inertes Lösungsmittel verwendet wird.

14. Verfahren gemäss Anspruch 8, dadurch gekennzeichnet, dass der Ameisensäureester der Formel III in äquimolarer Menge oder im Ueberschuss, bezogen auf den Aconitsäuretriester, eingesetzt wird.

15. 2-Oxo-2H-Pyran-4,5-dicarbonsäurediester der Formel V

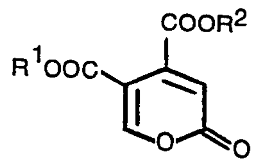

(V),

worin $R^1$ und $R^2$ unabhängig voneinander unsubstituierte oder substituierte aliphatische Kohlenwasserstoffreste darstellen.

16. Verfahren zur Herstellung von Pyranen der Formel V gemäss Anspruch 15, dadurch gekennzeichnet, dass man einen Alkoxymethylenaconitsäuretriester der Formel I

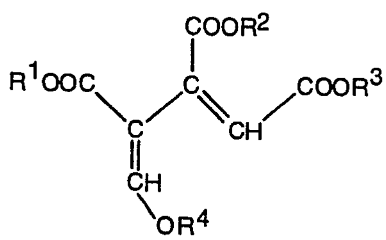

(I),

worin $R^1$, $R^2$, $R^3$ und $R^4$ unabhängig voneinander unsubstituierte oder substituierte aliphatische Kohlenwasserstoffreste darstellen, in Gegenwart einer starken wasserfreien Säure bei erhöhter Temperatur in an sich bekannter Weise cyclisiert und die erhaltene Verbindung der Formel V in an sich bekannter Weise isoliert.

17. Verwendung der 2-Oxo-2H-Pyran-4,5-dicarbonsäurediester gemäss Anspruch 15 als Dienkomponente in Diels-Alder-Reaktionen.

**Revendications**

1. Triesters d'acides alcoxyméthylène-aconitiques de formule I ci-dessous:

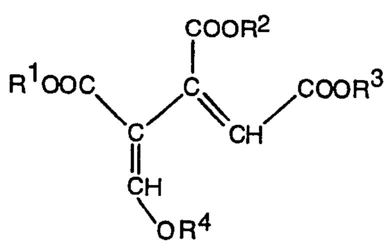

(I),

dans laquelle $R^1$, $R^2$, $R^3$ et $R^4$ désignent, indépendamment les uns des autres, des radicaux hydrocarbonés aliphatiques avec ou sans sustituents.

2. Esters selon la revendication 1, caractérisés en ce que les radicaux $R^1$ à $R^4$ sont, indépendamment les uns des autres, des alkyles linéaires ou ramifiés, des cycloalkyles, alkylcycloalkyles, aralkyles ou alcaralkyles.

3. Esters selon la revendication 2, caractérisés en ce que les radicaux $R^1$ à $R^4$ sont, indépendamment les uns des autres, des alkyles en $C_1$—$C_{18}$, des cycloalkyles en $C_3$—$C_{12}$, des alkylcycloalkyles en $C_4$—$C_{24}$, des aralkyles en $C_7$—$C_{15}$ ou des alcaralkyles en $C_8$—$C_{15}$.

4. Esters selon la revendication 1, caractérisés en ce que les radicaux hydrocarbonés aliphatiques $R^1$ à $R^4$ ont comme substituants, indépendamment les uns des autres, un groupe alcoxy ou nitrile ou bien un ou plusieurs atomes d'halogènes.

5. Esters selon la revendication 1, caractérisés en ce que les radicaux $R^1$ à $R^4$ sont des alkyles en $C_1$—$C_6$, indépendamment les uns des autres.

6. Esters selon la revendication 1, caractérisés en ce que les radicaux $R^1$ à $R^4$ sont identiques.

7. Esters selon la revendication 1, caractérisés en ce que les radicaux $R^1$ à $R^4$ sont le groupe méthyle ou éthyle, indépendamment les uns des autres.

8. Procédé de préparation des triesters d'acides alcoxyméthylène-aconitiques de formule I selon la revendication 1 par réaction de composés carbonyliques à liaisons CH acides avec un ester en présence de $TiCl_4$, d'une amine tertiaire et d'un solvant formant un complexe, procédé caractérisé en ce que l'on fait réagir comme composé carbonylique un triester de l'acide aconitrique de formule II:

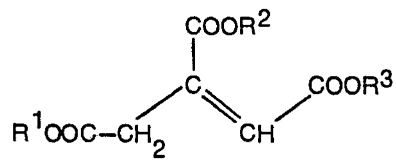

(II),

$R^1$, $R^2$ et $R^3$ étant, indépendamment les uns des autres, des radicaux hydrocarbonés aliphatiques avec ou sans substituants, avec comme ester un ester de l'acide formique de formule III:

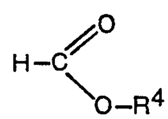

(III),

$R^4$ ayant, indépendamment, la même signification que $R^1$.

9. Procédé selon la revendication 8, caractérisé en ce que l'on effectue la réaction entre −20°C et +50°C.

10. Procédé selon la revendication 8, caractérisé en ce que l'amine tertiaire est une amine de formule IV:

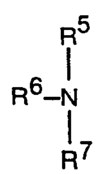

(IV),

(dans laquelle $R^5$, $R^6$ et $R^7$ représentent, indépendamment les uns des autres, des alkyles linéaires ou ramifiés, des cycloalkyles, des aralkyles ou des aryles), ou bien une amine hétéroaromatique ou aliphatico-hétérocyclique, cette dernière étant alkylée sur l'azote.

11. Procédé selon la revendication 10, caractérisé en ce que l'amine tertiaire est la N-méthyl-morpholine.

12. Procédé selon la revendication 8, caractérisé en ce que le solvant formant un complexe est un éther.

13. Procédé selon la revendication 8, caractérisé en ce que l'on effectue la réaction en présence d'un solvant inerte supplémentaire.

14. Procédé selon la revendication 8, caractérisé en ce que l'on emploie pour la réaction la proportion équimolaire ou un excès de l'ester formique de formule III par rappôrt au triester de l'acide aconitique.

15. Diesters d'acide 2-oxo-2H-pyranne-4,5-dicarboxylique de formule V:

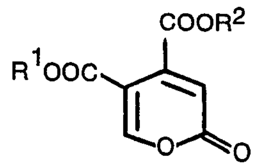

(V),

dans laquelle $R^1$ et $R^2$ représentent, indépendamment les uns des autres, des radicaux hydrocarbonés aliphatiques avec ou sand substituants.

16. Procédé de préparation des pyrannes de formule V selon la revendication 15, procédé caractérisé en ce que l'on cyclise de manière conue à chaud un triester d'acide alcoxyméthylène-aconitique de formule I:

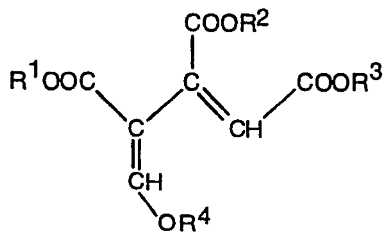

(I),

dans laquelle $R^1$, $R^2$, $R^3$ et $R^4$ représentent, indépendamment les uns des autres, des radicaux hydrocarbonés aliphatiques avec ou sans substituants, en présence d'un acide fort anhydre, puis on isole de manière connue le composé de formule V formé.

17. Emploi des diesters d'acide 2-oxo-2H-pyranne-4,5-dicarboxylique selon la revendication 15 comme diènes dans des réactions de Diels-Alder.

**Claims**

1. An alkoxymethyleneaconitic acid triester of formula I

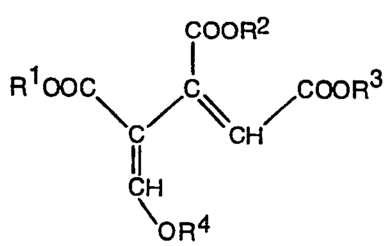

(I),

in which $R^1$, $R^2$, $R^3$ and $R^4$ are each independently of one another unsubstituted or substituted aliphatic hydrocarbon radicals.

2. An ester according to claim 1, wherein the radicals $R^1$, $R^2$, $R^3$ and $R^4$ are each independently of one another linear or branched alkyl, cycloalkyl, alkylcycloalkyl, aralkyl or alkaralkyl.

3. An ester according to claim 2, wherein the radicals $R^1$, $R^2$, $R^3$ and $R^4$ are each independently of one another $C_1$—$C_{18}$alkyl, $C_3$—$C_{12}$cycloalkyl, $C_4$—$C_{24}$alkylcycloalkyl, $C_7$—$C_{15}$aralkyl or $C_8$—$C_{15}$alkaralkyl.

4. An ester according to claim 1, wherein the aliphatic hydrocarbon radicals $R^1$, $R^2$, $R^3$ and $R^4$ are each independently of one another substituted by an alkoxy or nitrile group or by one or more halogen atoms.

5. An ester according to claim 1, wherein the aliphatic hydrocarbon radicals $R^1$, $R^2$, $R^3$ and $R^4$ are each independently of one another $C_1$—$C_6$alkyl.

6. An ester according to claim 1, wherein the aliphatic hydrocarbon radicals $R^1$, $R^2$, $R^3$ and $R^4$ are identical.

7. An ester according to claim 1, wherein the aliphatic hydrocarbon radicals $R^1$, $R^2$, $R^3$ and $R^4$ are each independently of one another methyl or ethyl.

8. A process for the preparation of an alkoxymethyleneaconitic acid triester of formula I according to claim 1 by reacting a carbonyl compound with CH acidic bonds and an ester in the presence of $TiCl_4$, a tertiary amine and a complexing solvent, which process comprises reacting, as carbonyl compound, an aconitic acid triester of formula II

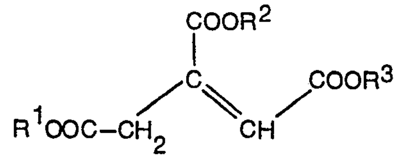
(II),

in which $R^1$, $R^2$ and $R^3$ are each independently of one another unsubstituted or substituted aliphatic hydrocarbon radicals, and, as ester, a formic acid ester of formula III

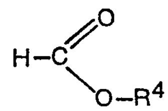
(III),

in which $R^4$ independently has the same meaning as $R^1$.

9. A process accordieR$^1$.

9. A process according to claim 8, wherein the reaction is carried out at a temperature from −20°C to +50°C.

10. A process according to claim 8, wherein the tertiary amine is an amine of formula IV

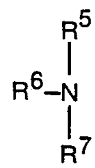
(IV),

in which $R^5$, $R^6$ and $R^7$ are each independently of one another branched or unbranched alkyl, cycloalkyl, aralkyl or aryl, or is a hetero-aromatic or aliphatic heterocyclic amine, aliphatic heterocyclic amines being N-alkylated.

11. A process according to claim 10, wherein the tertiary amine is N-methylmorpholine.

12. A process according to claim 8, wherein the complexing solvent is an ether.

13. A process according to claim 8, wherein an inert solvent is additionally employed in the reaction.

14. A process according to claim 8, wherein an equimolar amount of the formic acid ester of formula III or an excess thereof, based on the aconitic acid triester, is employed.

15. A 2-oxo-2H-pyran-4,5-dicarboxylic acid diester of formula V

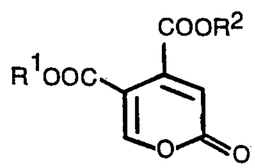
(V),

in which $R^1$ and $R^2$ are each independently of the other an unsubstituted or substituted aliphatic hydrocarbon radical.

16. A process for the preparation of a pyran of formula V according to claim 15, which comprises cyclising, in a manner known per se, an alkoxymethyleneaconitic acid triester of formula I

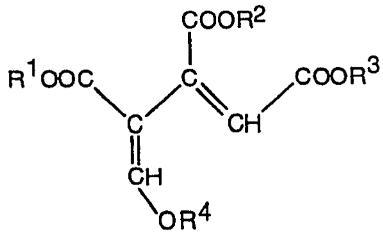

(I),

in which $R^1$, $R^2$, $R^3$ and $R^4$ are each independently of one another unsubstituted or substituted aliphatic hydrocarbon radicals, in the presence of a strong anhydrous acid at elevated temperature, and isolating the resultant compound of formula V in a manner known per se.

17. Use of a 2-oxo-2H-pyran-4,5-dicarboxylic acid diester according to claim 15 as diene component in Diels-Alder reactions.